(19) Europäisches Patentamt
European Patent Office
Office européen des brevets

(11) Veröffentlichungsnummer: **0 276 804 B1**

(12) **EUROPÄISCHE PATENTSCHRIFT**

(45) Veröffentlichungstag der Patentschrift: **18.03.92**

(51) Int. Cl.[5]: **C07D 285/16**, C07D 417/04, A61K 31/54

(21) Anmeldenummer: **88101055.7**

(22) Anmeldetag: **26.01.88**

Die Akte enthält technische Angaben, die nach dem Eingang der Anmeldung eingereicht wurden und die nicht in dieser Patentschrift enthalten sind.

# (54) 5-(3-Alkyl-5-tert.butyl-4-hydroxyphenyl)-2-amino-6H-1,3,4-thiadiazine, Verfahren zu ihrer Herstellung, die sie enthaltenden Arzneimittel und ihre Verwendung.

(30) Priorität: **30.01.87 DE 3702756**

(43) Veröffentlichungstag der Anmeldung: **03.08.88 Patentblatt 88/31**

(45) Bekanntmachung des Hinweises auf die Patenterteilung: **18.03.92 Patentblatt 92/12**

(84) Benannte Vertragsstaaten: **AT BE CH DE ES FR GB GR IT LI LU NL SE**

(56) Entgegenhaltungen:
**EP-A- 0 164 765**
**DE-A- 3 031 703**
**US-A- 4 272 532**

**CHEMICAL ABSTRACTS, Band 82, Nr. 9, 3. März 1975, Columbus, Ohio, USA; YOSHIDA, N.; NISHIMURA, T.; YASUDA, H.; (SANKYO, CO.) "2-Amino-1,3,4-thiadiazine derivatives." Seite 579, Spalte 1, Zusammenfassung-Nr. 57 744t**

**Chem. Abs., Bd. 90, Nr. 15 (1979) S. 28, 114920p**

**Chem. Abs., Bd. 104, Nr. 7 (1986), S. 513, 50789a**

(73) Patentinhaber: **HOECHST AKTIENGESELLSCHAFT**
**Postfach 80 03 20**
**W-6230 Frankfurt am Main 80(DE)**

(72) Erfinder: **Thorwart, Werner, Dr.**
**Am Gänsborn 3b**
**W-6203 Hochheim am Main(DE)**
Erfinder: **Gebert, Ulrich, Dr.**
**Albert-Lortzing-Strasse 2**
**W-6233 Kelkheim (Taunus)(DE)**
Erfinder: **Schleyerbach, Rudolf, Dr.**
**Finkenweg 10**
**W-6238 Hofheim am Taunus(DE)**
Erfinder: **Bartlett, Robert R., Dr.**
**Sandbergstrasse 20**
**W-6100 Darmstadt(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

5-(3-Alkyl-5-tert.butyl-4-hydroxyphenyl)-2-amino-6H-1,3,4-thiadiazine, Verfahren zu ihrer Herstellung, die sie enthaltenden Arzneimittel und ihre Verwendung.

Die vorliegende Erfindung betrifft neue 5-(3-Alkyl-5-tert.butyl-4-hydroxyphenyl)-2-amino-6H-1,3,4-thiadiazine, Verfahren zu ihrer Herstellung und ihre Verwendung als Wirkstoffe in Arzneimitteln zur Behandlung entzündlicher, insbesondere entzündlich rheumatischer Erkrankungen und von Schmerzzuständen.

Bei den bisher in der Rheumatherapie vorzugsweise eingesetzten nicht-steroidalen Antiphlogistika handelt es sich fast ausschließlich um verhältnismäßig starke Cyclooxygenase-Inhibitoren, die den endogenen Abbau der Arachidonsäure zu den entzündungs- und schmerzfördernden Prostaglandinen hemmen. Allerdings stehen im ursächlichen Zusammenhang mit einer zu starken Hemmung der Cyclooxygenase-Aktivität eine Reihe von gravierenden Nebenwirkungen, wie gastrointestinale Beschwerden, Nierenfunktionsstörungen und allergische Reaktionen (z.B. Hautallergien und Asthmaanfälle), die insbesondere bei der üblicherweise notwendigen Langzeitbehandlung häufig zum Abbruch der Therapie zwingen (vergl. K. Brune, Eur. J. Rheumatol. Inflam. 5 (1982), S. 335 - 349). Ein weiterer mit dem beschriebenen Wirkungsmechanismus ursächlich verbundener Nachteil dieser klassichen nichtsteroidalen Antiphlogistika besteht darin, daß sie zwar die Beseitigung oder Linderung der Symptome Schmerz, Entzündung und Schwellung gestatten, aber jene pathologischen Prozesse unbeeinflußt lassen, die den progredienten Verlauf der entzündlich rheumatischen Erkrankungen mitbedingen.

Es besteht somit ein dringendes Bedürfnis an therapeutisch nutzbaren Antirheumatika, die sich aufgrund eines günstigeren Wirkungsprofils von den bekannten nichtsteroidalen Antiphlogistika vorteilhaft durch bessere Verträglichkeit einerseits und einen mehr gegen den progredienten Verlauf des rheumatischen Krankheitsgeschehens gerichteten Eingriff andererseits unterscheiden. Erfolgversprechende Ansatzpunkte hierfür bieten solche antiphlogistisch und analgetisch wirksamen Pharmaka, die verstärkt in den alternativen Weg des Arachidonsäureabbaus eingreifen, indem sie beispielsweise die 5-Lipoxygenase hemmen und somit die exzessive Bildung per proinflammatorischen Leukotriene unterbinden und die hochreaktiven Sauerstoffradikale, die als Entzündungsmediatoren die Zell- und Gewebszerstörung in den entzündlich rheumatischen Gelenken perpetuell unterhalten, desaktivieren und damit die Möglichkeit zum medikamentösen Eingriff in den Circulus vitiosus der rheumatischen Erkrankungen eröffnen.

Überraschend wurde nun gefunden, daß durch Einführung bestimmter 3-Alkyl-5-tert.butyl-4-hydroxyphenyl-Reste in die 5-Stellung von gegebenenfalls in 6-Position substituierten 2-Amino-6H-1,3,4-thiadiazinen neuartige Verbindungen erhalten werden, die aufgrund ihrer pharmakologischen Eigenschaften die zuvor aufgestellten Anforderungen erfüllen und sich demnach hervorragend zur Behandlung rheumatischer Erkrankungen eignen.

Die auch gastral gut verträglichen Verbindungen hemmen im Unterschied zu den bekannten nicht-steroidalen Antiphlogistika das Arachidonsäure-abbauende Enzym 5-Lipoxygenase bereits in therapeutisch relevanten Dosen. Die Fähigkeit der Verbindungen, Sauerstoffradikale zu desaktivieren, zeigt sich beispielsweise im Modell der ®Adriamycin (Fa. Farmitalia)-induzierten Entzündung.

In der Literatur sind bereits mehrfach 5-phenylierte 2-Amino-6H-1,3,4-thiadiazine beschrieben worden (P. K. Bose, Quart. J. Ind. Chem. Soc. 2 (1925), S. 95 - 114; H. Beyer et al., Liebigs Ann. Chem. 741 - (1970), S. 45 - 54; H. Beyer, Quart. Rep. Sulfur Chem. 5 (1970), S. 177 - 189. R. R. Schmidt und H. Huth, THL 1975,S. 33 - 36; J. Y. Postovskii et al., Khim. Geterotsikl. Soedin. 1976, S. 1051 - 1055, ref. in Chem. Abstr. Vol. 85 (1976), S. 521 Ref. Nr. 85:177376h;W.D. Pfeiffer et al., Z. Chem. 17 (1977), S. 218 - 220, und R. E. Busby und T. W. Dominey, J. Chem. Soc. 1980, S. 890 - 899), von denen jedoch keine pharmakologischen Eigenschaften bekannt sind. Demgegenüber wird in der DE-OS 30 31 703 über bestimmte 5-(Chlorphenyl)-2-amino-6H-1,3,4-thiadiazine berichtet, denen eine krampf- und angstlösende Wirkung zugeschrieben wird. Ferner werden in der DD-PS 220 311 unter anderem 5-Phenyl-6H-1,3,4-thiadiazine mit dem sehr speziellen N-Methyl-N-(1-hydroxy-1-phenylprop-2-yl)-amino-Rest in 2-Stellung offenbart, die spasmolytische Aktivität besitzen sollen.

Die vorliegende Erfindung betrifft demgegenüber neue 2-Amino-6H-1,3,4-thiadiazine, die in 5-Stellung einen 3-Alkyl-5-tert.butyl-4-hydroxyphenyl-Rest als essentielle pharmakophore Gruppe und gegebenenfalls in 6-Position einen weiteren Substituenten tragen. Aufgrund ihrer zuvor erwähnten pharmakologischen Eigenschaften eignen sich die erfindungsgemäßen Verbindungen zur Verwendung in Arzneimitteln, insbesondere in solchen, die bei entzündlich rheumatischen Erkrankungen und Schmerzzuständen indiziert sind.

Die Japanische Druckschrift JA-PS 74-88889 beschreibt am Phenylrest einfach substituierte 5-phenylierte-2-Amino-6H-1,3,4-thiadiazine, die eine antivirale, entzündungshemmende oder schmerzlindernde Wirkung aufweisen.

Gegenstand der Erfindung sind somit 5-(3-Alkyl-5-tert.butyl-4-hydroxyphenyl)-2-amino-6H-1,3,4-thiadiazine der allgemeinen Formel I, in der

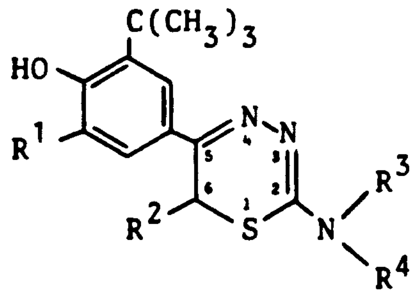

(I)

| | |
|---|---|
| $R^1$ | eine geradkettige oder verzweigte Alkylgruppe mit 1 bis 4 C-Atomen und |
| $R^2$ | eine Wasserstoffatom oder einen Alkylrest mit 1 bis 3 C-Atomen darstellen, |
| $R^3$ und $R^4$ | unabhängig voneinander für eine geradkettige oder verzweigte $(C_1-C_6)$Alkyl-, $(C_3-C_6)$-Alkenyl- oder $(C_3-C_6)$Alkinylgruppe stehen mit Ausnahme der Inamine oder Enamine, die mit Phenyl oder mit Halogen, $(C_1-C_3)$Alkyl- oder $(C_1-C_3)$Alkoxy- substituiertem Phenyl, Hydroxy oder $(C_1-C_4)$Acyloxy substituiert und/oder deren C-C-Folge durch ein Heteroatom in Form von Sauerstoff oder Schwefel unterbrochen sein können, wobei einer der beiden Reste auch ein Wasserstoffatom bedeuten kann, oder |
| $R^3$ und $R^4$ | zusammen mit dem Stickstoffatom, an das sie gebunden sind, einen 4- bis 7-gliedrigen Ring bilden, der ein- oder mehrfach mit $(C_1-C_4)$Alkyl substituiert sein kann und/oder anstelle eines der ringständigen C-Atome ein weiteres Heteroatom in Form von Sauerstoff, Schwefel oder Stickstoff enthält, wobei letzterer anstelle von Wasserstoff auch eine Alkylgruppe mit 1 bis 3-C-Atomen oder einen Phenylrest tragen kann, |

sowie ihre physiologisch verträglichen Säureadditionssalze.

Bevorzugt sind dabei solche Verbindungen der Formel I und deren Salze, bei denen $R^1$ tert.Butyl oder Methyl bedeutet oder $R^2$ für Wasserstoff oder Methyl steht. Bevorzugt sind auch solche Verbindungen, bei denen $R^3$ und $R^4$ unabhängig voneinander für eine geradkettige oder verzweigte $(C_1-C_3)$Alkyl-oder $(C_3-C_4)$-Alkenylgruppe stehen mit Ausnahme der Enamine, die mit ggf. substituiertem Phenyl oder Hydroxy substituiert oder deren C-C-Folge durch ein Sauerstoffatom unterbrochen sein können, wobei einer der beiden Reste auch ein Wasserstoffatom bedeuten kann, oder $R^3$ und $R^4$ zusammen mit dem Stickstoffatom, an das sie gebunden sind, einen 5- oder 6-gliedrigen Ring bilden, der mit einem $(C_1-C_2)$Alkylrest substituiert sein kann oder anstelle eines der ringständigen C-Atome ein weiteres Heteroatom in Form von Sauerstoff oder Stickstoff enthält, wobei letzterer anstelle von Wasserstoff auch eine Alkylgruppe mit 1 oder 2 C-Atomen tragen kann. Unter diesen Verbindungen sind wiederum jene besonders hervorzuheben, in denen $R^1$ einen tert.Butylrest darstellt und gleichzeitig $R^2$ für Wasserstoff oder Methyl steht und $R^3$ und $R^4$ unabhängig voneinander eine geradkettige oder verzweigte $(C_1-C_3)$Alkyl- oder $(C_3-C_4)$Alkenylgruppe mit Ausnahme der Enamine, die mit Phenyl substituiert sein können, bedeuten, wobei einer der beiden Reste auch ein Wasserstoffatom sein kann, oder $R^3$ und $R^4$ zusammen mit dem Stickstoffatom, an das sie gebunden sind, einen Piperidin-, 4-Methylpiperidin-, Piperazin- oder 4-Methylpiperazin-Ring bilden.

Als Alkylreste für die Gruppe $R^1$ kommen Methyl, Äthyl, n-Propyl, Isopropyl, n-Butyl, Isobutyl, sek.Butyl und tert.Butyl, für die Gruppe $R^2$ Methyl, Äthyl, n-Propyl und Isopropyl und für die Gruppen $R^3$ und/oder $R^4$ Methyl, Äthyl, Propyl, Isopropyl, n-Butyl, Isobutyl, sek.Butyl, tert.Butyl und die verschiedenen Pentyl- und Hexylreste, z. B. Neopentyl, in Frage. Weitere geeignete Gruppen für die Reste $R^3$ und/oder $R^4$ sind beispielsweise Allyl, die verschiedenen Butenyl-, Pentenyl- und Hexenylreste, Propargyl, die verschiedenen Butinyl-, Pentinyl- und Hexinylreste, 2-Hydroxyäthyl,Meth-und Äthoxyäthyl, Methyl- und Äthylthioäthyl, ggf. substituiertes Benzyl und Phenäthyl sowie unter Einschluß des Stickstoffatoms , an das sie gebunden sind, die cyclischen Systeme, wie Pyrrolidin, Piperidin, 4-Methylpiperidin, Morpholin, Thiomorpholin, Piperazin, 4-Methyl- und 4-Phenylpiperazin.

Gegenstand der Erfindung ist ferner ein Verfahren zur Herstellung der neuen 5-(3-Alkyl-5-tert.butyl-4-hydroxyphenyl)-2-amino-6H-1,3,4-thiadiazine gemäß Formel I und ihrer physiologisch verträglichen Säureadditionssalze, das darin besteht, daß man ein 2-Halogen-1-phenylalkanon der Formel II

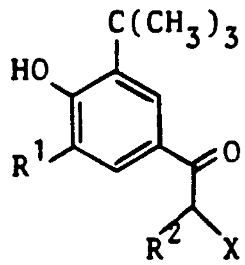

(II)

mit einem Thiosemicarbazid der Formel III,

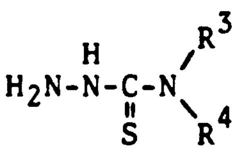

(III)

wobei $R^1$, $R^2$, $R^3$ und $R^4$ die voranstehend genannten Bedeutungen haben und X für ein Halogenatom, vorzugweise Chlor oder Brom, steht, zu den erfindungsgemäßen Verbindungen der Formel I umsetzt und diese entweder in freier Form isoliert oder mit geeigneten Säuren in physiologisch verträgliche Additionssalze umwandelt.

Für die Herstellung von Säureadditionssalzen kommen beispielsweise Mineralsäuren, wie Bromwasserstoff-, Chlorwasserstoff-, Schwefel- oder Phosphorsäure; organische Säuren, wie Essig-, Milch-, Malein-, Fumar-, Oxal-, Wein-, Zitronen- oder Glukonsäure; oder andere physiologisch verträgliche Säuren, wie Sulfonsäuren, z.B. Benzolsulfonsäure, p-Toluolsulfon-, Methansulfon-, Trifluormethylsulfon- und Cyclohexylamidosulfonsäure, in Frage.

Die für das einstufige Verfahren als Ausgangsstoffe verwendeten 2-Halogen-1-phenylalkanone der Formel II sind literaturbekannt oder lassen sich aus den 1-(3-Alkyl-5-tert.butyl-4-hydroxyphenyl)alkanonen durch Umsetzung mit einem geeigneten Halogenierungsmittel nach den im Houben-Weyl Bd. V/4 (1960), S. 171 - 189, beschriebenen Methoden leicht herstellen.

Als geeignete Verbindungen II seien beispielsweise das 2-Brom-1-(3,5-di-tert.butyl-4-hydroxyphenyl)-äthanon und 2-Brom-1-(5-tert.butyl-3-methyl-4-hydroxyphenyl)-äthanon genannt, die sich durch Halogenierung der entsprechend substituierten 1-Phenylalkanone mit elementarem Brom oder mit Kupfer-II-bromid nach einem Verfahren von L. C. King und G. K. Ostrum, J. Org. Chem. 29 (1964), S. 3459 - 3461, herstellen lassen.

Zur Gewinnung jener Verbindung der Formel II, in denen für X ein Chloratom steht, eignet sich insbesondere elementares Chlor oder Sulfurylchlorid, wobei letzteres vorzugsweise bei Temperaturen zwischen etwa 10 und 30 ° C in Anwesenheit inerter Lösungsmittel, wie etwa Methylenchlorid oder Chloroform, mit den entsprechenden 1-Phenylalkanonen zur Reaktion gebracht wird. Ein weiteres Herstellungsverfahren besteht in der Friedel-Crafts-Acylierung von 2-Alkyl-6-tert.butylphenolen mit vorzugsweise Chloracetylchlorid in Gegenwart von Lewissäuren, wie z. B. Aluminiumchlorid oder Bortrifluorid.

Die ebenfalls als Ausgangsstoffe eingesetzten 4-mono- oder 4,4-disubstiutierten Thiosemicarbazide der Formel III sind meist literaturbekannt oder können nach den in Houben-Weyl, Bd. E 4, S. 506 - 515, und von K. A. Jensen et al., Acta Chem. Scand, 22 (1968), S. 1 - 50, beschriebenen Methoden dargestellt werden. So lassen sich die Thiosemicarbazide III, bei denen einer der Reste $R^3$ oder $R^4$ Wasserstoff darstellt, vorteilhaft durch Addition von Hydrazin an Isothiocyanate gewinnen, während die 4,4-disubstituierten Thiosemicarbazide III vorzugsweise durch Umsetzung der entsprechend N,N-disubstituierten Thiocarbamidisäurechloride mit Hydrazin hergestellt werden. Zur Vermeidung störender Nebenreaktionen wird dabei vorteilhaft in einem aprotischen Lösungsmittel, wie beispielsweise Chloroform, Tetrachlormethan oder Diäthyläther bei Reaktionstemperaturen unterhalb etwa 10 ° C gearbeitet (D.L. Klayman et al., J. Med. Chem. 22 (1979), S. 1367 - 1373). Ein weiteres Verfahren, das sowohl die Herstellung der 4-mono- als auch der 4,4-disubstituierten Thiosemicarbazide gestattet, geht von den entsprechend N-substituierten Thiocarbamoylmercaptoessigsäuren der Formel IV aus,

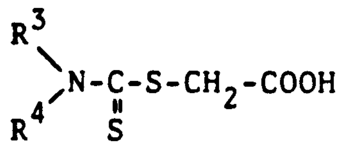

(IV)

die sich mit Hydrazin in Gegenwart verdünnter Basen, wie z. B. Natrium- oder Kaliumhydroxid, bei bis zu mehrtägigem Erhitzen unter Rückfluß in die Verbindungen III umwandeln lassen (K. A. Jensen, J. prakt. Chem. 159 (1941), S. 189 - 192).

Die Umsetzung der 2-Halogen-1-phenylalkanone II mit den Thiosemicarbaziden III wird zweckmäßig mit äquimolaren Mengen der beiden Reaktanten in einem gegenüber den Reaktionspartnern inerten Lösungs- oder Verteilungsmittel durchgeführt. Hierfür kommen insbesondere niedere Alkohole, wie Methanol, Äthanol, n-Propanol, Isopropanol und die verschiedenen Butanole, oder Essigsäureäthylester sowie deren Gemische, vorzugsweise jedoch Äthanol in Frage. Die Umsetzung erfolgt in der Regel bei Temperaturen zwischen etwa -20°C und der Siedetemperatur des jeweils verwendeten Reaktionsmediums, bevorzugt jedoch bei etwa 20 bis 70°C. Die Reaktionszeit kann je nach Reaktivität der Reaktionspartner, der Art des Reaktionsmediums und der angewendeten Reaktionstemperatur zwischen etwa 5 Minuten und 2 Stunden liegen. Gewöhnlich kristallisieren die Endprodukte der Formel I beim langsamen Abkühlen der Reaktionsmischung analysenrein aus, so daß sich im allgemeinen weitere Reinigungsoperationen erübrigen. Bleibt jedoch die spontane Kristallisation aus, so empfiehlt es sich, das Rekationsgemisch unter vermindertem Druck vollständig einzudampfen und den zurückbleibenden Rückstand in einem geeigneten Lösungs- bzw. Verteilungsmittel, beispielsweise einem Carbonsäureester, wie Essigsäureäthylester; einem Keton, wie Aceton oder Methyläthylketon; einem Äther, wie Diisopropyläther oder Methyl-tert.butyläther; oder deren Gemischen, bei Raumtemperatur ausgiebig zu digerieren. Das dabei anfallende kristalline Produkt wird entweder durch Umkristallisation oder durch nochmaliges Digerieren in demselben Lösungs- bzw. Verteilungsmittel unter kurzzeitigem Aufkochen, Abkühlen, Absaugen und Trocknen im Vakuumschrank gereinigt.
Die Darstellung der erfindungsgemäßen Verbindungen I mit einem Wasserstoffatom in der Position von $R^3$ oder $R^4$ aus den 4-monosubstituierten Thiosemicarbaziden III verläuft dann besonders einheitlich, wenn diese in Form ihrer Salze, beispielsweise ihrer Hydrochloride, zur Reaktion eingesetzt werden.

Die erfindungsgemäßen 5-(3-Alkyl-5-tert.butyl-4-hydroxyphenyl)-2-amino-6H-1,3,4-thiadiazine der Formel I und ihre physiologisch verträglichen Säureadditionssalze eignen sich aufgrund ihrer wertvollen pharmakologischen Eigenschaften bei gleichzeitig hervorragender Verträglichkeit in besonderer Weise für die Verwendung als Wirkstoffe in Arzneimitteln, insbesondere in solchen zur Behandlung von entzündlich rheumatischen Erkrankungen und Schmerzzuständen. Sie können entweder für sich allein, beispielsweise in Form von Mikrokapseln, in Mischungen untereinander oder in Kombination mit geeigneten Hilfs- und/oder Trägerstoffen verabreicht werden.

Gegenstand der Erfindung sind somit auch Arzneimittel, die aus mindestens einer Verbindung der Formel I, gegebenenfalls in Form eines ihrer Säureadditionssalze, bestehen oder mindestens einen dieser Wirkstoffe neben pharmazeutisch geeigneten und physiologisch verträglichen Trägerstoffen, Verdünnungsmitteln und/oder anderen Hilfsstoffen enthalten.

Die erfindungsgemäßen Arzneimittel können oral, topisch, rektal oder gegebenenfalls auch parenteral appliziert werden, wobei die orale Anwendung bevorzugt ist.
Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung üblicherweise Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyäthylenglykole und Lösungsmittel, wie etwa steriles Wasser und ein- oder mehrwertige Alkohole, z. B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung gemäß Formel I und/oder mindestens eines entsprechenden Säureadditionssalzes enthält. Bei festen Dosierungseinheiten, wie Tabletten, Kapseln, Dragees oder Suppositorien, kann diese Dosis bis zu etwa 800 mg, bevorzugt jedoch etwa 100 bis 500 mg betragen.

Für die Behandlung eines an entzündlich rheumatischen Erkrankungen und Schmerzzuständen leidenden, erwachsenen Patienten sind - je nach Wirksamkeit der Verbindungen gemäß Formel I und/oder der

entsprechenden Säureadditionssalze am Menschen - Tagesdosen von etwa 100 bis 200 mg Wirkstoff, vorzugsweise etwa 300 bis 1000 mg, bei oraler Verbreichung indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Verbindungen der Formel I und die entsprechenden Säureadditionssalze bei der Herstellung der vorgenannten galenischen Zubereitungsformen auch zusammen mit anderen geeigneten Wirkstoffen, beispielsweise Antiuricopathika, Thrombocytenaggregationshemmern, anderen Analgetika und anderen steroidalen oder nicht-steroidalen Antiphlogistika, formuliert werden.

Die Strukturaller nachstehend beschriebenen Verbindungen wurde durch Elementaranalyse und IR- sowie $^{1}$H-NMR-Spektren gesichert. Die gemäß den nachfolgenden Beispielen 1 bis 4 und die auf analoge Weise hergestellten Verbindungen der Formel I sind in Tabelle 1 zusammengefaßt.

Beispiel 1: 5-(3,5-Di-tert.butyl-4-hydroxyphenyl)-2-dimethylamino-6H-1,3,4-thiadiazin-hydrobromid

a) 2-Brom-1-(3,5-di-tert.butyl-4-hydroxyphenyl)-äthanon

206 g (0,83 mol) 1-(3,5-Di-tert.butyl-4-hydroxyphenyl)-äthanon wurden unter Rühren in 415 ml Methylenchlorid gelöst, zum Sieden erhitzt und innerhalb von 30 Minuten tropfenweise mit 144 g (0,9 mol) Brom versetzt. Danach wurde für weitere 2 Stunden unter Rückfluß erhitzt, die Mischung abgekühlt, mit 400 ml Wasser versetzt, die organische Phase abgetrennt und über Natriumsulfat getrocknet. Nach dem Entfernen des Lösungsmittels unter vermindertem Druck wurde das erhaltene feste Rohprodukt aus 540 ml Methylcyclohexan umkristallisiert.

Ausbeute: 191 g (67 % der Theorie)
Schmelzpunkt: 105 - 108° C
$C_{16}H_{23}BrO_2$ (MG = 327,3)

b) 5-(3,5-Di-tert.butyl-4-hydroxyphenyl)-2-dimethylamino-6H-1,3,4-thiadiazin-hydrobromid

196,4 g (0,6 mol) 2-Brom-1-(3,5-di-tert.butyl-4-hydroxyphenyl)-äthanon aus Stufe a) und 59,6 g (0,5 mol) 4,4-Dimethylthiosemicarbazid wurden in 1500 ml Äthanol suspendiert und unter Rühren langsam auf 70° C erhitzt, wobei eine klare Lösung entstand. Man ließ bei dieser Temperatur 10 Minuten nachrühren, kühlte dann ab und entfernte das Lösungsmittel unter vermindertem Druck. Ausrühren des als Öl angefallenen Rückstandes mit 500 ml Essigsäureäthylester ergab einen Kristallbrei, der nochmals in 500 ml Essigsäureäthylester kurzzeitig zum Sieden erhitzt wurde. Das kristalline Produkt wurde abfiltriert, mit 150 ml Essisäureäthylester gewaschen und im Vakuumschrank getrocknet.

Ausbeute: 165,5 g (77,3 % der Theorie)
Schmelzpunt: 256 - 257° C
$C_{19}H_{30}BrN_3OS$ (MG = 428,4)
Analyse: Berechnet: C 53,27 %; H 7,06 %; Br 18,65 %; N 9,81 %; S 7,48 %
Gefunden: C 53,29 %; H 7,11 %; Br 18,10 %; N 9,77 %; S 7,60 %

Beispiel 2: 5-(3,5-Di-tert.butyl-4-hydroxyphenyl)-2-(4-methylpiperidino)-6H-1,3,4-thiadiazin-hydrobromid

a) (4-Methylpiperidino)-thiocarbonsäurehydrazid

Unter Eiskühlung und Rühren tropfte man innerhalb von 2 Stunden eine Lösung von 35,5 g (0,2 mol) (4-Methylpiperidino)-thiocarbonylchlorid in 70 ml Chloroform zu 100,1 g 99 %igem Hydrazinhydrat in 50 ml Chloroform. Nach 2-stündigem Nachrühren wurde das Reaktionsgemisch unter vermindertem Druck zur Trockne eingedampft und der ölige Rückstand in 200 ml Diisopropyläther ausgerührt. Die dabei gebildeten Kristalle löste man aus Essigsäureäthylester um.

Ausbeute: 21,5 g (62 % der Theorie)
Schmelzpunkt:L 113 - 115° C
$C_7H_{15}N_3S$ (MG = 173,3)

b) 5-(3,5-Di-tert.butyl-4-hydroxyphenyl)-2-(4-methylpiperidino)-6H-1,3,4-thiadiazin-hydrobromid

18,0 g (0,055 mol) 2-Brom-1-(3,5-di-tert.butyl-4-hydroxyphenyl)-äthanon des Beispiels 1a) und 8,7 g

(0,05 mol) (4-Methylpiperidino)-thiocarbonsäurehydrazid aus Stufe a) wurden in 150 ml Äthanol gelöst und 10 Minuten zum Sieden erhitzt. Die Aufarbeitung erfolgte unter den im Beispiel 1b) beschriebenen Bedingungen.

Ausbeute: 17,4 g ( 72 % der Theorie)
Schmelzpunkt: 190 - 193° C
$C_{23}H_{36}BrN_3OS$ (MG = 482,5)
Analyse: Berechnet: C 57,26 %; H 7,52 %; Br 16,56 %; N 8,71 %; S 6,65 %
Gefunden: C 57,02 %; H 7,61 %; Br 16,51 %; N 8,73 %; S 6,77 %

Beispiel 3: 5-(5-tert.Butyl-3-methyl-4-hydroxyphenyl)-2-dimethylamino-6H-1,3,4-thiadiazin-hydrobromid

a) 2-Brom-1-(5-tert.butyl-3-methyl-4-hydroxyphenyl)-äthanon

Eine zum Sieden erhitzte Suspension von 179 g (0,8 mol) Kupfer(II)bromid in 360 ml Essigsäureäthylester wurde unter Rühren tropfenweise mit einer Lösung von 82,5 g (0,4 mol) 1-(5-tert.Butyl-3-methyl-4-hydroxyphenyl)-äthanon in 360 ml Chloroform versetzt. Anschließend wurde bis zur Beendigung der Bromwasserstoff-Entwicklung 4 Stunden lang unter Rückfluß erhitzt. Nach dem Abkühlen auf Raumtemperatur wurden die Kupfersalze abgesaugt, der Filterrückstand mehrmals mit Essigsäureäthylester gewaschen, das Filtrat unter vermindertem Druck eingeengt und der feste Rückstand aus Cyclohexan umkristallisiert.

Ausbeute: 81,9 g (72 % der Theorie)
Schmelzpunkt: 90 - 92 °C
$C_{13}H_{17}BrO_2$ (MG = 285,2)

b) 5-(5-tert.Butyl-3-methyl-4-hydroxyphenyl)-2-dimethylamino-6H-1,3,4-thiadiazin-hydrobromid

Eine Lösung von 11,7 g (0,041 mol) 2-Brom-1-(5-tert.butyl)-3-methyl-4-hydroxyphenyl)-äthanon aus Stufe a) und 4,4 g (0,037 mol) 4,4-Dimethylthiosemicarbazid in 130 ml Äthanol wurde kurzzeitig unter Rückfluß erhitzt. Nach dem Erkalten wurde das Reaktionsgemisch entsprechend Beispiel 1b) aufgearbeitet.

Ausbeute: 12,2 g (85,3 % der Theorie)
Schmelzpunkt: 197° C
$C_{16}H_{24}BrN_3OS$ (MG = 386,4)
Analyse: Berechnet: C 49,74 %; H 6,26 %; Br 20,68 %; N 10,87 %; S 8,30 %
Gefunden: C 49,37 %; H 6,21 %; Br 20,49 %; N 10,87 % S 8,00 %

Beispiel 4: 5-(3,5-Di-tert.butyl-4-hydroxyphenyl)-2-methylamino-6H-1,3,4-thiadiazin-hydrochlorid

a) 2-Chlor-1-(3,5-di-tert.butyl-4-hydroxyphenyl)-äthanon

Zu einer Lösung von 19,9 g (0,08 mol) 1-(3,5-Di-tert.butyl-4-hydroxyphenyl)-äthanon in 250 ml Methylenchlorid tropfte man unter Rühren bei Raumtemperatur 16,2 g (0,12 mol) Sulfurylchlorid in 50 ml Methylenchlorid hinzu. Nach 3-stündigem Nachrühren bei Raumtemperatur wurde der Ansatz zuerst mit Wasser und dann mit einer gesättigten Natriumhydrogencarbonatlösung ausgeschüttelt, über Natriumsulfat getrocknet und unter vermindertem Druck eingeengt. Der Rückstand wurde aus 60 ml Isopropanol umkristallisiert.

Ausbeute: 12,7 g (56 % der Theorie)
Schmelzpunkt: 120 - 122° C
$C_{16}H_{23}ClO_2$ (MG = 282,8)
Analyse: Berechnet; C 67,95 %; H 8,20 %; Cl 12,54 %
Gefunden: C 67,87 %; H 8,32 %; Cl 12,29 %

b) 5-(3,5-Di-tert.butyl-4-hydroxyphenyl)-2-methylamino-6H-1,3,4-thiadiazin-hydrochlorid

10,7 g (0,038 mol) 2-Chlor-1-(3,5-di-tert.butyl-4-hydroxyphenyl)-äthanon aus Stufe a) und 4,8 g (0,034 mol) 4-Methylthiosemicarbazid-hydrochlorid wurden in 120 ml Äthanol gelöst und 30 Minuten zum Sieden erhitzt. Nach dem Erkalten wurde das Lösungsmittel unter vermindertem Druck abdestilliert und der harzige Rückstand in 60 ml Essigsäureäthylester warm gelöst. Beim Stehenlassen fiel alsbald das Produkt kristallin aus, das abfiltriert und nochmals aus 150 ml eines Gemisches von Essigsäureäthylester und Methylenchlorid (10 : 1) umkristallisiert wurde.

Ausbeute: 8,9 g (71 % der Theorie)
Schmelzpunkt: 207 - 209° C
$C_{18}H_{28}ClN_3OS$ (MG = 370,0)
Analyse: Berechnet: C 58,44 %; H 7,63 %; Cl 9,58 %; N 11,36 %; S 8,67 %
Gefunden: C 58,13 %; H 7,72 %; Cl 9,31 %; N 11,22 %; S 8,78 %

Tabelle 1: Verbindungen gemäß Formel I (siehe Anspruch 1)

| Beispiel | $R^1$ | $R^2$ | $R^3$ | $R^4$ | Schmelzpunkt °C |
|---|---|---|---|---|---|
| 1 | $(H_3C)_3C$- | H | $-CH_3$ | $-CH_3$ | 256 - 257 (Hydrobromid) |
| 2 | $(H_3C)_3C$- | H | 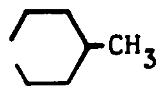 | | 190 - 193 (Hydrobromid) |
| 3 | $H_3C$- | H | $-CH_3$ | $-CH_3$ | 197 (Hydrobromid) |
| 4 | $(H_3C)_3C$- | H | H | $-CH_3$ | 207 - 209 (Hydrochlorid) |
| 5 | $H_3C$- | H | H | $-CH_2-CH=CH_2$ | 198 - 200 (Hydrobromid) |
| 6 | $H_3C$- | H | $-CH_2-CH_3$ | $-CH_2-CH_3$ | 182 - 184 (Hydrobromid) |
| 7 | $H_3C$- | H | $-CH_3$ | $-CH_2$- 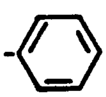 | 190 - 191 (Hydrobromid) |
| 8 | $(H_3C)_3C$- | H | $-CH_3$ | $-CH_2-CH_3$ | 200 (Hydrobromid) |
| 9 | $(H_3C)_3C$- | H | $-CH_2-CH_3$ | $-CH_2-CH_3$ | 188 - 190 (Hydrobromid) |
| 10 | $(H_3C)_3C$- | H | $-CH_2-CH_3$ | $-CH(CH_3)_2$ | 179 - 181 (Hydrobromid) |
| 11 | $(H_3C)_3C$- | H | $-CH_2-CH_2-CH_3$ | $-CH_2-CH_2-CH_3$ | 187 - 189 (Hydrobromid) |
| 12 | $(H_3C)_3C$- | H | H | $-CH_2$- 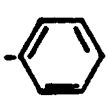 | 223 - 225 (Hydrobromid) |

Tabelle 1 (Fortsetzung)

| Beispiel | $R^1$ | $R^2$ | $R^3$ | $R^4$ | Schmelzpunkt °C |
|---|---|---|---|---|---|
| 13 | $(H_3C)_3C-$ | H | $-CH_3$ | $-CH_2-$ 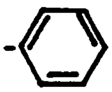 | 192 - 194 (Hydrobromid) |
| 14 | $(H_3C)_3C-$ | H | H | $-CH_2-CH=CH_2$ | 193 - 194 (Hydrobromid) |
| 15 | $(H_3C)_3C-$ | H | H | $-CH_2-CH_2-OH$ | 214 - 215 (Hydrobromid) |
| 16 | $(H_3C)_3C-$ | H | $-CH_2-CH_2-O-CH_3$ | $-CH_2-CH_2-O-CH_3$ | 151 - 152 (Hydrochlorid) |
| 17 | $(H_3C)_3C-$ | H | 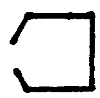 | | 247 - 250 (Hydrobromid) |
| 18 | $(H_3C)_3C-$ | H | 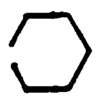 | | 177 - 179 (Hydrobromid) |
| 19 | $(H_3C)_3C-$ | H | 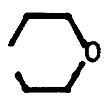 | | 188 - 191 (Hydrobromid) |
| 20 | $(H_3C)_3C-$ | H | 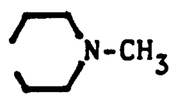 | | 280 (Zers.) (Dihydrobromid) |
| 21 | $(H_3C)_3C-$ | H | 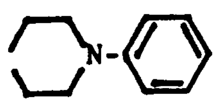 | | 188 - 190 (Hydrobromid) |
| 22 | $(H_3C)_3C-$ | $H_3C-$ | $-CH_3$ | $-CH_3$ | 199- 200 (Hydrobromid) |

Tabelle 1 (Fortsetzung)

| Beispiel | $R^1$ | $R^2$ | $R^3$ | $R^4$ | Schmelzpunkt °C |
|---|---|---|---|---|---|
| 23 | $(H_3C)_3C-$ | H | H | $-CH_2-C(CH_3)_3$ | 227 - 229 (Hydrobromid) |
| 24 | $(H_3C)_3C-$ | H | H | $-CH_2-CH_2-O-CH_3$ | 197 - 198 (Hydrobromid) |
| 25 | $(H_3C)_3C-$ | H | H | $-CH_2-$ 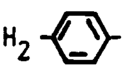 $-OCH_3$ | 208 - 209 (Hydrobromid) |
| 26 | $(H_3C)_3C-$ | H | H | $-CH_2-$ 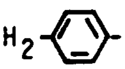 $-Cl$ | 219 - 220 (Hydrobromid) |
| 27 | $(H_3C)_3C-$ | H | H | $-CH_2-$ 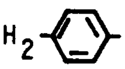 $-CH_3$ | 220 - 221 (Hydrobromid) |
| 28 | $(H_3C)_3C-$ | H | $-CH_2-CH_2-OH$ | $-CH_2-$ 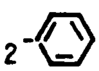 | 183 - 184 (Hydrobromid) |
| 29 | $(H_3C)_3C-$ | H | H | $-CH_2-CH_2-$ 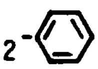 | 217 - 218 (Hydrobromid) |
| 30 | $(H_3C)_3C-$ | $H_3C-$ | H | $-CH_2-CH=CH_2$ | 195 - 196 (Hydrobromid) |
| 31 | $(H_3C)_3C-$ | $H_3C-$ | $-CH_3$ | $-CH_2-$ 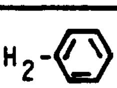 | 146 - 147 (Hydrobromid) |
| 32 | $(H_3C)_3C-$ | H | 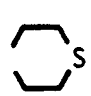 | | 190 - 191 (Hydrobromid) |

Pharmakologische Prüfung und Ergebnisse

Die Prüfung der erfindungsgemäßen Verbindungen der Formel I auf antiphlogistische und analgetische Wirkung, Sauerstoffradikale desaktivierende Potenz, ulzerogene Aktivität und akute Toxizität erfolgte in den anschließend beschriebenen Tiermodellen, wobei das in der Rheumatherapie zu den Standardpräparaten der ersten Wahl zählende Antiphlogistikum Naproxen (2-(6-Methoxy-2-naphthyl)-propionsäure) als Vergleichssubstanz in die Untersuchungen miteinbezogen wurde.

1. Adjuvans-Arthritis

Die Untersuchungen wurden nach der Methode von Pearson (Arthrit. Rheum. 2 (1959), S. 44) durchgeführt. Als Versuchstiere dienten männliche Ratten eines Wistar-Lewis-Stammes mit einem Körpergewicht zwischen 130 und 200 g. Die zu prüfenden Verbindungen wurden in Dosen von 50 mg pro kg Körpergewicht einmal täglich vom 1. bis zum 5. Versuchstag oral (p. o.) appliziert. Die Tiere einer Kontrollgruppe erhielten nur das Vehikel. Jede Präparat- und die Kontrollgruppe umfaßte 8 Tiere. Als Wirkungskriterium diente die prozentuale Herabsetzung der Pfotenvolumenzunahme gegenüber jener der unbehandelten Kontrollgruppe.

2. Akute gastrale Ulzerogenität

Die Untersuchung erfolgte an jeweils 10 männlichen Sprague-Dawley Ratten mit durch Hungerstreß sensibilisierter Magenschleimhaut. Das Körpergewicht der Tiere lag zwischen 200 und 300 g. 48 Stunden vor Applikation der Prüfpräparate bis zum Töten der Tiere wurde bei freiem Zugang zum Trinkwasser das Futter entzogen. Die Ratten wurden 24 Stunden nach oraler Substanzgabe getötet, die Mägen entnommen, unter fließendem Wasser gereinigt und auf Schleimhautläsionen inspiziert. Als Ulzera galten alle makroskopisch sichtbaren Läsionen.

Bestimmt wurde die Anzahl der Tiere mit Ulzera je Dosis und daraus die $UD_{50}$-Werte, also jene Dosen, mit denen bei 50 % der Tiere Läsionen hervorgerufen wurden, nach Litchfield und Wilcoxon (J. Pharmacol. exp. Ther. 96 (1949),S. 99) berechnet.

3. Akute Toxizität

Die Bestimmung der $LD_{50}$-Werte erfolgte standardgemäß über die innerhalb von 7 Tagen bei NMRI-(Naval Medical Research Institute)-Mäusen (6 Tiere pro dosi) nach einmaliger intraperitonealer (i. p.) Gabe auftretende Mortalität.

Die Ergebnisse dieser Untersuchungen, die die Überlegenheit der erfindungsgemäßen Verbindungen der Formel I gegenüber dem Standardpräparat Naproxen eindeutig belegen, sind in der nachstehenden Tabelle 2 zusammengefaßt.

Tabelle 2:

| Antiphlogistische Wirkung, Ulzerogenität und akute Toxizität | | | |
|---|---|---|---|
| Verbindung aus Beispiel | Adjuvans-Arthritis (% Hemmung bei 50 mg/kg p.o.) | Akute Ulzerogenität $UD_{50}$ (mg/kg) | Akute Toxizität $LD_{50}$ (mg/kg) |
| 1 | 69 *) | 200 | 600 - 1200 |
| 2 | 62 | 100 | 600 - 1200 |
| 3 | 58 | > 200 | 600 - 1200 |
| 4 | 81 | 100 - 200 | 300 - 600 |
| 8 | 61 *) | > 200 | 300 - 600 |
| 9 | 55 *) | > 200 | 600 - 1200 |
| 10 | 82 | 200 | > 1200 |
| 11 | 54 | 100 - 200 | > 1200 |
| 12 | 65 | > 200 | 600 - 1200 |
| 13 | 71 | > 200 | 600 - 1200 |
| 14 | 75 | > 200 | 600 - 1200 |
| 18 | 74 | 100 | > 1200 |
| 20 | 72 | 200 | 150 - 300 |
| 22 | 84 *) | > 200 | 600 - 1200 |
| Naproxen | 55 | 23 | 500 |

*) % Hemmung bei 12,5 mg/kg p.o.

Aus der Dosiswirkungskurve im Modell der Adjuvans-Arthritis ergab sich beispielsweise für die Verbindung aus Beispiel 1 ein $ED_{50}$-Wert von 6,0 mg/kg, der deutlich günstiger als der entsprechende Vergleichswert von 17,5 mg/kg für das Standardpräparat Naproxen liegt. Bezogen auf die akute Ulzerogenität errechnet sich daraus durch die Quotientenbildung von $UD_{50}$ zu $ED_{50}$ eine therapeutische Breite von etwa 33 für die Verbindung des Beispiels 1, die beim Vergleichspräparat Naproxen nur 1,3 beträgt, womit besonders eindrucksvoll unterstrichen wird, welch hoher Stellenwert der außerordentlich guten gastralen Verträglichkeit der erfindungsgemäßen Verbindungen zukommt. Eine ebenso eindeutige Überlegenheit gegenüber der Vergleichsverbindung ergibt sich, wenn man bei der Berechnung der therapeutischen Breite die ermittelten $LD_{50}$-Werte zugrundelegt und den Quotienten $LD_{50}/ED_{50}$ bildet, der für die Verbindung des Beispiels 1 um 150 und für Naproxen bei 28,6 liegt.

Auch in weiteren speziellen Versuchen erwiesen sich die erfindungsgemäßen Verbindungen dem Standardpräparat Naproxen eindeutig überlegen.

4. Antioxydative Wirkung

Nach heutiger Lehrmeinung sind an dem multifaktoriell bedingten, progressiven Verlauf der rheumatoiden Arthritis und anderer entzündlicher Erkrankungen aggressive Sauerstoffradikale maßgeblich beteiligt, die während des chronischen Entzündungsprozesses exzessiv gebildet werden und selbst als hochtoxische Entzündungsmediatoren die über eine irreversible Lipidperoxydation der Zellmembranen verlaufende Bindegewebszerstörung perpetuell unterhalten. Folglich sollten antioxydativ wirksame Pharmaka mit der Fähigkeit zur Desaktivierung dieser extrem cytotoxischen Sauerstoffradikale einen gezielten Eingriff in das chronische Entzündungsgeschehen gestatten. Ein geeignetes Tiermodell für einer derartige durch Sauerstoffradikale vermittelte Gewebszerstörung stellt die an der Ratte mit Adriamycin (Doxorubicin) induzierte Entzündung dar.

Die Untersuchungen wurden nach der Methode von D.M. Siegel et al. (Inflammation 4 (1980), S. 233) an männlichen Sprague-Dawley Ratten mit einem Körpergewicht zwischen 200 und 230 g in Gruppen von jeweils 7 Tieren durchgeführt, die 0,1 mg Adriamycin, gelöst in 0,1 ml 0,9%iger Kochsalzlösung, durch

subkutane Injektion in die linke Hinterpfote erhielten.
72 Stunden danach wurde die Pfotenvolumenzunahme als Maß für den Entzündungsgrad durch plethysmographische Messung bestimmt.
Die orale Verabreichung der Prüfpräparate in 1%iger wäßriger Carboxymethylcellulose-Suspension erfolgte einmal täglich an 4 aufeinanderfolgenden Tagen, beginnend mit dem Tag der Adriamycin-Injektion.

Wie aus Tabelle 3 hervorgeht, zeigten auch in diesem Test beispielsweise die Verbindungen aus Beispiel 1 und 14 einen starken, dosisabhängigen Schutzeffekt gegen die durch Adriamycin ausgelöste Gewebszerstörung. Sowohl steroidale als auch nicht-steroidale Antiphlogistika, einschließlich Naproxen, sind in dieser Versuchsanordnung unwirksam.

Tabelle 3:

| Hemmung der Adriamycin-induzierten Entzündung | | | |
|---|---|---|---|
| Tier kollektiv | Dosis in mg/kg p.o. | Pfotenvolumenzunahme in µl | Schutzwirkung in % |
| Kontrolle | - | 390 | - |
| Verbindung aus Beispiel 1 | 25<br>50 | 350<br>120 | 10<br>69 * |
| Verbindung aus Beispiel 14 | 25<br>50 | 250<br>160 | 36<br>59 * |

* Signifikanz $p < 0{,}05$

5. Hemmung der 5-Lipoxygenase

Die Hemmwirkung der erfindungsgemäßen Verbindungen auf den durch die 5-Lipoxygenase katalysierten Arachidonsäure-Abbau wurde, wie üblich, in In-vitro-Versuchen an isolierten polymorphkernigen Humangranulozyten nachgewiesen. Hierzu inkubierte man die Zellen bei 37° C mit dem jeweiligen Prüfpräparat und löste nach 5 Minuten mit dem Calcium-Ionophor A 23 187 (Calbiochem GmbH, Frankfurt/Main, BRD, Biochemical and Immunochemical Catalog 1985, S. 284) die Bildung des proinflammatorisch wirkenden Leukotrien $B_4$ ($LTB_4$) aus. Die in einem Zeitraum von 10 Minuten freigesetzte Menge an $LTB_4$ wurde nach Auftrennung durch Hochdruckflüssigkeitschromatographie (HPLC) mit Hilfe eines UV-Detektors quantitativ bestimmt.

Wie die nachstehende Tabelle 4 zeigt, ließ sich in dieser Versuchsanordnung die Bildung von $LTB_4$ und demzufolge der Arachidonsäure-Abbau über die 5-Lipoxygenase durch die erfindungsgemäßen Verbindungen der Formel I signifikant hemmen.

Tabelle 4:

| Hemmung der 5-Lipoxygenase | |
|---|---|
| Verbindung aus Beispiel | $IC_{50}$ ($10^{-6}$ mol/l) |
| 1 | 9 |
| 3 | 4 |
| 8 | 13 |
| 17 | 10 |

6. Analgetische Wirkung

Die Prüfung der erfindungsgemäßen Verbindungen der Formel I auf analgetische Wirksamkeit erfolgte im Essigsäure-Strecktest an der Maus nach der Methode von R. Koster et al., Fed. Prod. 18 (1959), S. 412. Als Versuchstiere dienten weibliche Mäuse eines NMRI-Stammes mit einem Körpergewicht (KG) zwischen 21 und 28 g. Gruppen von je 12 Tieren erhielten 0,1 ml/10 g KG einer 0,6 %igen Essigsäurelösung intraperitoneal injiziert. Die Prüfsubstanzen wurden 30 Minuten vorher verabreicht. Unmittelbar nach der

Essigsäure-Injektion wurden die Tiere einzeln gesetzt und die innerhalb 15 Minuten auftretenden typischen Streckbewegungen gezählt, die in einem kurzen Anspannen der Bauchmuskulatur mit Einziehen der Flankenpartien und anschließendem Strecken des Hinterkörpers und mindestens einer Hinterextremität bestehen.
Zur Beurteilung des analgetischen Effektes wurde die Anzahl der Streck-Reaktionen in Beziehung zu jener einer unbehandelten Kontrollgruppe gesetzt, wobei jene Tiere, die weniger als die Hälfte der durchschnittlich von den Kontrolltieren ausgeführten Streckbewegungen zeigten, als analgesiert gewertet wurden. Die Prüfsubstanzen wurden oral in einem Volumen von 10 ml/kg KG in 1 %iger wäßriger Carboxymethylcellulose(CMC)-Suspension appliziert.

Die Testergebnisse in der nachstehenden Tabelle 5 belegen, daß die erfindungsgemäßen Verbindungen der Formel I auch eine starke analgetische Wirkungskomponente aufweisen.

Tabelle 5:

| Analgetische Wirkung im Essigsäurestrecktest an der Maus | |
|---|---|
| Verbindung aus Beispiel | Analgesierte Tiere in % nach einer oralen Dosis von 158 mg/kg |
| 1 | 83 |
| 2 | 75 |
| 3 | 42 |
| 4 | 83 |
| 8 | 92 |
| 13 | 42 |
| 14 | 67 |
| 22 | 92 |

**Patentansprüche**

**Patentansprüche für folgende Vertragsstaaten : AT, BE, CH, DE, FR, GB, IT, LI, LU, NL, SE**

**1.** 5-(3-Alkyl-5-tert.butyl-4-hydroxyphenyl)-2-amino-6H-1,3,4-thiadiazine der allgemeinen Formel I

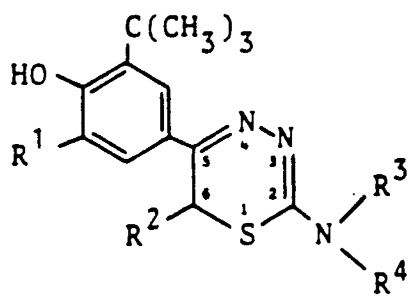

(I),

in der

- $R^1$ eine geradkettige oder verzweigte Alkylgruppe mit 1 bis 4 C-Atomen und
- $R^2$ ein Wasserstoffatom oder einen Alkylrest mit 1 bis 3 C-Atomen darstellen,
- $R^3$ und $R^4$ unabhängig voneinander für eine geradkettige oder verzweigte $(C_1-C_6)$Alkyl-, $(C_3-C_6)$-Alkenyl- oder $(C_3-C_6)$Alkinylgruppe stehen mit Ausnahme der Inamine oder Enamine, die mit Phenyl oder mit Halogen, $(C_1-C_3)$Alkyl- oder $(C_1-C_3)$Alkoxy-substituiertem Phenyl, Hydroxy oder $(C_1-C_4)$Acyloxy substituiert und/oder deren C-C-Folge durch ein Heteroatom in Form von Sauerstoff oder Schwefel unterbrochen sein können, wobei einer der beiden Reste auch ein Wasserstoffatom bedeuten kann, oder
- $R^3$ und $R^4$ zusammen mit dem Stickstoffatom, an das sie gebunden sind, einen 4- bis 7-gliedrigen Ring bilden, der ein- oder mehrfach mit $(C_1-C_4)$Alkyl substituiert sein kann und/oder anstelle eines der ringständigen C-Atome ein weiteres Heteroatom in Form

von Sauerstoff, Schwefel oder Stickstoff enthält, wobei letzterer anstelle von Wasserstoff auch eine Alkylgruppe mit 1 bis 3-C-Atomen oder einen Phenylrest tragen kann, sowie ihre physiologisch verträglichen Säureadditionssalze.

**2.** Verbindungen nach Anspruch 1, gekennzeichnet durch wenigstens eines der Merkmale, daß $R^1$ tert.Butyl oder Methyl bedeutet oder $R^2$ für Wasserstoff oder Methyl steht oder $R^3$ und $R^4$ unabhängig voneinander für eine geradkettige oder verzweigte $(C_1\text{-}C_3)$Alkyl- oder $(C_3\text{-}C_4)$Alkenylgruppe stehen mit Ausnahme der Enamine, die mit ggf. substituiertem Phenyl oder Hydroxy substituiert oder deren C-C-Folge durch ein Sauerstoffatom unterbrochen sein können, wobei einer der beiden Reste auch ein Wasserstoffatom bedeuten kann, oder $R^3$ und $R^4$ zusammen mit dem Stickstoffatom, an das sie gebunden sind, einen 5- oder 6-gliedrigen Ring bilden, der mit einem $(C_1\text{-}C_2)$Alkylrest substituiert sein kann oder anstelle eines der ringständigen C-Atome ein weiteres Heteroatom in Form von Sauerstoff oder Stickstoff enthält, wobei letzterer anstelle von Wasserstoff auch eine Alkylgruppe mit 1 oder 2 C-Atomen tragen kann.

**3.** Verbindungen nach Anspruch 1 oder 2, dadurch gekennzeichnet, daß $R^1$ einen tert.Butylrest darstellt und gleichzeitig $R^2$ für Wasserstoff oder Methyl steht und $R^3$ und $R^4$ unabhängig voneinander eine geradkettige oder verzweigte $(C_1\text{-}C_3)$Alkyl- oder $(C_3\text{-}C_4)$Alkenylgruppe mit Ausnahme der Enamine, die mit Phenyl substituiert sein können, bedeuten, wobei einer der beiden Reste auch ein Wasserstoffatom sein kann, oder $R^3$ und $R^4$ zusammen mit dem Stickstoffatom, an das sie gebunden sind, einen Piperidin-, 4-Methylpiperidin-, Piperazin- oder 4-Methylpiperazin-Ring bilden.

**4.** Verfahren zur Herstellung von 5-(3-Alkyl-5-tert.butyl-4-hydroxyphenyl)-2-amino-6H-1,3,4-thiadiazinen der Formel I und ihren physiologisch verträglichen Säureadditionssalzen gemäß einem oder mehreren der Ansprüche 1 bis 3, dadurch gekennzeichnet,daß man ein 2-Halogen-1-phenylalkanon der Formel II

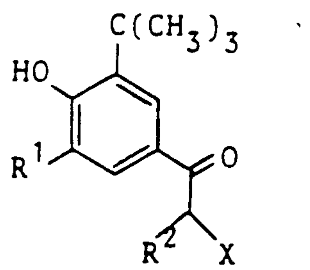

(II)

mit einem Thiosemicarbazid der Formel III,

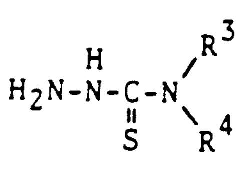

(III)

wobei $R^1$, $R^2$, $R^3$ und $R^4$ die im Anspruch 1 genannten Bedeutungen haben und X für ein Halogenatom steht, zu den Verbindungen der Formel I umsetzt und diese entweder in freier Form isoliert oder mit geeigneten Säuren in physiologisch verträgliche Additionssalze umwandelt.

**5.** Verbindungen der Formel I sowie deren physiologisch verträgliche Säureadditionssalze gemäß einem oder mehreren der Ansprüche 1 - 3 zur Anwendung als Heilmittel.

**6.** Arzneimittel, gekennzeichnet durch einen Gehalt an - oder bestehend aus - mindestens einer Verbindung der Formel I und/oder einem ihrer physiologisch verträglichen Säureadditionssalze nach einem

oder mehreren der Ansprüche 1 bis 3 hergestellten Verbindung.

**7.** Arzneimittel nach Anspruch 6 zur Vorbeugung und Behandlung von Krankheiten, bei denen die therapeutische Anwendung von Entzündungshemmern, Analgetika, Sauerstoffradikale desaktivierenden Mitteln und/oder Hemmstoffen des durch die 5-Lipoxygenase vermittelten Arachidonsäureabbaus indiziert ist.

**8.** Arzneimittel nach Anspruch 6 oder 7 zur Vorbeugung und Behandlung von entzündlichen, insbesondere entzündlich rheumatischen Erkrankungen und/oder Schmerzzuständen.

**9.** Verfahren zur Herstellung eines Arzneimittels gemäß einem oder mehreren der Ansprüche 6 - 8, dadurch gekennzeichnet, daß man mindestens eine Verbindung der Formel I und/oder mindestens ein physiologisch verträgliches Säureadditionssalz einer solchen Verbindung mit einem physiologisch annehmbaren Träger und gegebenenfalls weiteren Zusatz- und/oder Hilfsstoffen in eine geeignete Darreichungsform bringt.

**10.** Verwendung von Verbindungen der Formel I und/oder deren physiologisch verträglichen Säureadditionssalzen zur Herstellung von Arzneimitteln gemäß den Ansprüchen 7 und 8.

**Patentansprüche für folgende Vertragsstaaten : ES, GR**

**1.** Verfahren zur Herstellung von 5-(3-Alkyl-5-tert.butyl-4-hydroxyphenyl)-2-amino-6H-1,3,4-thiadiazine der allgemeinen Formel I

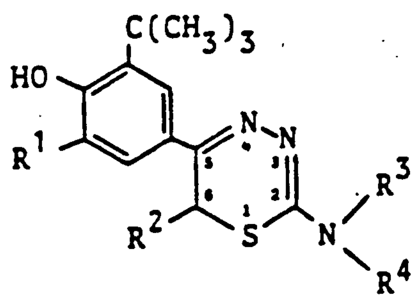

(I),

in der

| | |
|---|---|
| $R^1$ | eine geradkettige oder verzweigte Alkylgruppe mit 1 bis 4 C-Atomen und |
| $R^2$ | ein Wasserstoffatom oder einen Alkylrest mit 1 bis 3 C-Atomen darstellen, |
| $R^3$ und $R^4$ | unabhängig voneinander für eine geradkettige oder verzweigte $(C_1-C_6)$Alkyl-, $(C_3-C_6)$-Alkenyl- oder $(C_3-C_6)$Alkinylgruppe stehen mit Ausnahme der Inamine oder Enamine, die mit Phenyl oder mit Halogen, $(C_1-C_3)$Alkyl- oder $(C_1-C_3)$Alkoxy-substituiertem Phenyl, Hydroxy oder $(C_1-C_4)$Acyloxy substituiert und/oder deren C-C-Folge durch ein Heteroatom in Form von Sauerstoff oder Schwefel unterbrochen sein können, wobei einer den beiden Reste auch ein Wasserstoffatom bedeuten kann, oder |
| $R^3$ und $R^4$ | zusammen mit dem Stickstoffatom, an das sie gebunden sind, einen 4- bis 7-gliedrigen Ring bilden, der ein- oder mehrfach mit $(C_1-C_4)$Alkyl substituiert sein kann und/oder anstelle eines der ringständigen C-Atome ein weiteres Heteroatom in Form von Sauerstoff, Schwefel oder Stickstoff enthält, wobei letzterer anstelle von Wasserstoff auch eine Alkylgruppe mit 1 bis 3-C-Atomen oder einen Phenylrest tragen kann, |

sowie ihrer physiologisch verträglichen Säureadditionssalze, dadurch gekennzeichnet, daß man ein 2-Halogen-1-phenylalkanon der Formel II

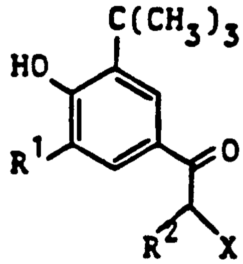

(II)

mit einem Thiosemicarbazid der Formel III,

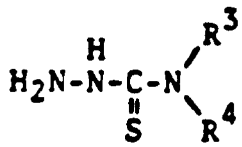

(III)

wobei $R^1$, $R^2$, $R^3$ und $R^4$ die bei Formel I genannten Bedeutungen haben und X für ein Halogenatom steht, zu den Verbindungen der Formel I umsetzt und diese entweder in freier Form isoliert oder mit geeigneten Säuren in physiologisch verträgliche Additionssalze umwandelt.

**2.** Verfahren nach Anspruch 1, dadurch gekennzeichnet, daß man Verbindungen der Formel I mit wenigstens einem der Merkmale herstellt, daß $R^1$ tert.Butyl oder Methyl bedeutet oder $R^2$ für Wasserstoff oder Methyl steht oder $R^3$ und $R^4$ unabhängig voneinander für eine geradkettige oder verzweigte $(C_1\text{-}C_3)$Alkyl-oder $(C_3\text{-}C_4)$Alkenylgruppe stehen mit Ausnahme der Enamine, die mit ggf. substituiertem Phenyl oder Hydroxy substituiert oder deren C-C-Folge durch ein Sauerstoffatom unterbrochen sein können, wobei einer der beiden Reste auch ein Wasserstoffatom bedeuten kann, oder $R^3$ und $R^4$ zusammen mit dem Stickstoffatom, an das sie gebunden sind, einen 5- oder 6-gliedrigen Ring bilden, der mit einem $(C_1\text{-}C_2)$Alkyrest substituiert sein kann oder anstelle eines der ringständigen C-Atome ein weiteres Heteroatom in Form von Sauerstoff oder Stickstoff enthält, wobei letzterer anstelle von Wasserstoff auch eine Alkylgruppe mit 1 oder 2 C-Atomen tragen kann.

**3.** Verfahren nach Anspruch 1 oder 2, dadurch gekennzeichnet, daß man Verbindungen der Formel I herstellt, worin $R^1$ einen tert.Butylrest darstellt und gleichzeitig $R^2$ für Wasserstoff oder Methyl steht und $R^3$ und $R^4$ unabhängig voneinander eine geradkettige oder verzweigte $(C_1\text{-}C_3)$Alkyl- oder $(C_3\text{-}C_4)$-Alkenylgruppe mit Ausnahme der Enamine, die mit Phenyl substituiert sein können, bedeuten, wobei einer der beiden Reste auch ein Wasserstoffatom sein kann, oder $R^3$ und $R^4$ zusammen mit dem Stickstoffatom, an das sie gebunden sind, einen Piperidin-, 4-Methylpiperidin-, Piperazin- oder 4-Methylpiperazin-Ring bilden.

**4.** Verfahren zur Herstellung der Verbindungen der Formel I sowie deren physiologisch verträglichen Säureadditionssalzen gemäß einem oder mehreren der Ansprüche 1 - 3 zur Anwendung als Heilmittel.

**5.** Verfahren zur Herstellung eines Arzneimittels, dadurch gekennzeichnet, daß man mindestens eine Verbindung der Formel I gemäß der Definition in Anspruch 1 und/oder mindestens ein physiologisch verträgliches Säureadditionssalze einer solchen Verbindung und/oder mindestens eine der nach dem Verfahren gemäß einem oder mehreren der Ansprüche 1 - 3 erhaltenen Verbindungen mit einem physiologisch annehmbaren Träger und gegebenenfalls weiteren Zusatz- und/oder Hilfsstoffen in eine geeignete Darreichungsform bringt.

**6.** Verfahren nach Anspruch 5, dadurch gekennzeichnet, daß das verfahrensgemäß hergestellte Arzneimittel zur Vorbeugung und Behandlung von Krankheiten bestimmt ist, bei denen die therapeutische

Anwendung von Entzündungshemmern, Analgetika, Sauerstoffradikale desaktivierenden Mitteln und/oder Hemmstoffen des durch die 5-Lipoxygenase vermittelten Arachidonsäureabbaus indiziert ist.

7. Verfahren nach Anspruch 5 oder 6, dadurch gekennzeichnet, daß das verfahrensgemäß hergestellte Arzneimittel zur Vorbeugung und Behandlung von entzündlichen, insbesondere entzündlich rheumatischen Erkrankungen und/oder Schmerzzuständen bestimmt ist.

8. Verwendung von Verbindungen der Formel I gemäß der Definition in Anspruch 1 oder wie erhalten nach einem oder mehreren der Ansprüche 1 - 3, und/oder von deren physiologisch verträglichen Säureadditionssalze zur Herstellung von Arzneimitteln zur Vorbeugung und Behandlung der in den Ansprüchen 6 und 7 genannten Krankheiten.

**Claims**

**Claims for the following Contracting States : AT, BE, CH, DE, FR, GB, IT, LI, LU, NL, SE**

1. A 5-(3-alkyl-5-tert.butyl-4-hydroxyphenyl)-2-amino-6H-1,3,4-thiadiazine of the formula I

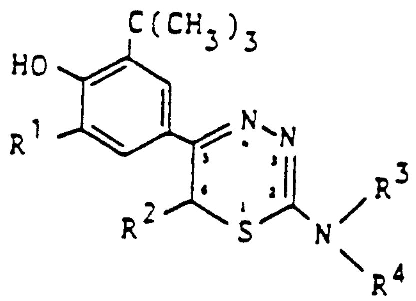

(I),

in which
$R^1$ denotes a straight-chain or branched alkyl group having 1 to 4 carbon atoms, and
$R^2$ denotes a hydrogen atom or an alkyl radical having 1 to 3 carbon atoms,
$R^3$ and $R^4$, independently of one another, represent a straight-chain or branched $(C_1-C_6)$alkyl, $(C_3-C_6)$-alkenyl or $(C_3-C_6)$alkynyl group, with the exception of inamines and enamines, which can be substituted by phenyl or halogen, $(C_1-C_3)$alkyl- or $(C_1-C_3)$alkoxy-substituted phenyl, hydroxyl or $(C_1-C_4)$acyloxy and/or whose C-C sequence may be interrupted by a heteroatom in the form of oxygen or sulfur, it also being possible for one of the two radicals to denote a hydrogen atom, or
$R^3$ and $R^4$, together with the nitrogen atom to which they are bound, form a 4- to 7-membered ring which may be monosubstituted or polysubstituted by $(C_1-C_4)$alkyl and/or contains, in place of one of the ring carbon atoms, a further heteroatom in the form of oxygen, sulfur or nitrogen, it also being possible for the latter to carry an alkyl group having 1 to 3 carbon atoms or a phenyl radical in place of hydrogen,
or a physiologically acceptable acid-addition salt thereof.

2. A compound as claimed in claim 1, which has at least one of the features that $R^1$ denotes tert.butyl or methyl or $R^2$ represents hydrogen or methyl or $R^3$ and $R^4$, independently of one another, represent a straight-chain or branched $(C_1-C_3)$alkyl or $(C_3-C_4)$alkenyl group, with the exception of enamines, which may be substituted by optionally substituted phenyl or hydroxyl or whose C-C sequence may be interrupted by an oxygen atom, it also being possible for one of the two radicals to denote a hydrogen atom, or $R^3$ and $R^4$, together with the nitrogen atom to which they are bound, form a 5- or 6-membered ring which may be substituted by a $(C_1-C_2)$alkyl radical or contains, in place of one of the ring carbon atoms, a further heteroatom in the form of oxygen or nitrogen, it also being possible for the latter to carry an alkyl group having 1 or 2 carbon atoms in place of hydrogen.

3. A compound as claimed in claim 1 or 2, wherein $R^1$ denotes a tert.butyl radical and simultaneously $R^2$ represents hydrogen or methyl and $R^3$ and $R^4$, independently of one another, denote a straight-chain or branched $(C_1-C_3)$alkyl or $(C_3-C_4)$alkenyl group, with the exception of enamines, which may be substituted by phenyl, it also being possible for one of the two radicals to be a hydrogen atom, or $R^3$

and $R^4$, together with the nitrogen atom to which they are bound, form a piperidine, 4-methylpiperidine, piperazine or 4-methylpiperazine ring.

**4.** A process for the preparation of a 5-(3-alkyl-5-tert.butyl-4-hydroxyphenyl)-2-amino-6H-1,3,4-thiadiazine of the formula I and a physiologically acceptable acid-addition salt thereof, as claimed in one or more of claims 1 to 3, which comprises reacting a 2-halo-1-phenylalkanone of the formula II

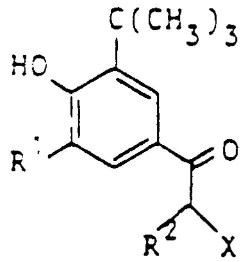

(II)

with a thiosemicarbazide of the formula III

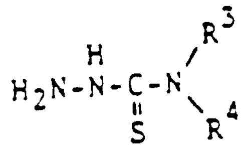

(III)

where $R^1$, $R^2$, $R^3$ and $R^4$ have the meanings mentioned in claim 1 and X represents a halogen atom, to give a compound of the formula I, and the latter being either isolated in free form or converted into a physiologically acceptable addition salt by means of a suitable acid.

**5.** A compound of the formula I, or a physiologically acceptable acid-addition salt thereof, as claimed in one or more of claims 1 - 3, for use as a medicament.

**6.** A medicament which contains - or comprises - at least one compound of the formula I and/or a physiologically acceptable acid-addition salt thereof, as claimed in one or more of claims 1 to 3.

**7.** A medicament as claimed in claim 6, for prevention and treatment of disorders in which the therapeutic use of inflammation inhibitors, analgesics, oxygen radical-deactivating agents and/or inhibitors of 5-lipoxygenase-induced degradation of arachidonic acid is indicated.

**8.** A medicament as claimed in claim 6 or 7, for prevention and treatment of inflammatory disorders, in particular inflammatory rheumatic disorders, and/or pain.

**9.** A process for the production of a medicament as claimed in one or more of claims 6 - 8, wherein at least one compound of the formula I and/or at least one physiologically acceptable acid-addition salt of such a compound is converted into a suitable form of administration by means of a physiologically acceptable excipient and, if appropriate, further additives and/or adjuvants.

**10.** The use of a compound of the formula I and/or a physiologically acceptable acid-addition salt thereof for the production of medicaments as claimed in claims 7 and 8.

**Claims for the following Contracting States : ES, GR**

**1.** A process for the preparation of a 5-(3-alkyl-5-tert.butyl-4-hydroxyphenyl)-2-amino-6H-1,3,4-thiadiazine of the formula I

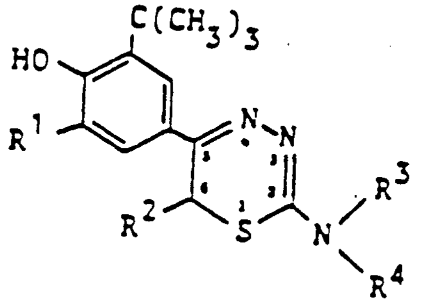

(I),

in which

$R^1$ denotes a straight-chain or branched alkyl group having 1 to 4 carbon atoms,

$R^2$ denotes a hydrogen atom or an alkyl radical having 1 to 3 carbon atoms,

$R^3$ and $R^4$, independently of one another, represent a straight-chain or branched $(C_1$-$C_6)$alkyl, $(C_3$-$C_6)$-alkenyl or $(C_3$-$C_6)$alkynyl group, with the exception of inamines or enamines, which can be substituted by phenyl or halogen, $(C_1$-$C_3)$alkyl- or $(C_1$-$C_3)$alkoxy-substituted phenyl, hydroxyl or $(C_1$-$C_4)$acyloxy and/or whose C-C sequence may be interrupted by a heteroatom in the form of oxygen or sulfur, it also being possible for one of the two radicals to denote a hydrogen atom, or

$R^3$ and $R^4$, together with the nitrogen atom to which they are bound, form a 4- to 7-membered ring which may be monosubstituted or polysubstituted by $(C_1$-$C_4)$alkyl and/or contains, in place of one of the ring carbon atoms, a further heteroatom in the form of oxygen, sulfur or nitrogen, it also being possible for the latter to carry an alkyl group having 1 to 3 carbon atoms or a phenyl radical in place of hydrogen,

or a physiologically acceptable acid-addition salt thereof, which comprises reacting a 2-halo-1-phenylalkanone of the formula II

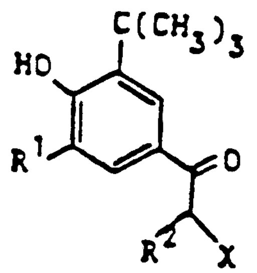

. (II)

with a thiosemicarbazide of the formula III

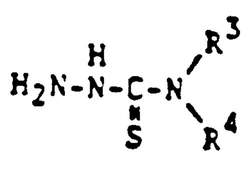

(III)

where $R^1$, $R^2$, $R^3$ and $R^4$ have the meanings mentioned in formula I and X represents a halogen atom, to give a compound of the formula I, and the latter being either isolated in free form or converted into a physiologically acceptable addition salt by means of a suitable acid.

**2.** The process as claimed in claim 1, wherein a compound of the formula I is prepared having at least one of the features that $R^1$ denotes tert.butyl or methyl or $R^2$ represents hydrogen or methyl or $R^3$ and $R^4$, independently of one another, represent a straight-chain or branched $(C_1$-$C_3)$alkyl or $(C_3$-$C_4)$alkenyl group, with the exception of enamines, which may be substituted by optionally substituted phenyl or hydroxyl or whose C-C sequence may be interrupted by an oxygen atom, it also being possible for one of the two radicals to denote a hydrogen atom, or $R^3$ and $R^4$, together with the nitrogen atom to which

they are bound, form a 5- or 6-membered ring which may be substituted by a $(C_1-C_2)$alkyl radical or contains, in place of one of the ring carbon atoms, a further heteroatom in the form of oxygen or nitrogen, it also being possible for the latter to carry an alkyl group having 1 or 2 carbon atoms in place of hydrogen.

**3.** The process as claimed in claim 1 or 2, wherein a compound of the formula I is prepared in which $R^1$ denotes a tert.butyl radical and simultaneously $R^2$ represents hydrogen or methyl and $R^3$ and $R^4$, independently of one another, denote a straight-chain or branched $(C_1-C_3)$alkyl or $(C_3-C_4)$alkenyl group, with the exception of enamines, which may be substituted by phenyl, it also being possible for one of the two radicals to be a hydrogen atom, or $R^3$ and $R^4$, together with the nitrogen atom to which they are bound, form a piperidine, 4-methylpiperidine, piperazine or 4-methylpiperazine ring.

**4.** A process for the preparation of a compound of the formula I, or a physiologically acceptable acid-addition salt thereof, as claimed in one or more of claims 1 - 3 for use as a medicament.

**5.** A process for the production of a medicament, wherein at least one compound of the formula I as defined in claim 1 and/or at least one physiologically acceptable acid-addition salt of such a compound and/or at least one compound obtained by the process as claimed in one or more of claims 1 - 3 is converted into a suitable form of administration by means of a physiologically acceptable excipient and, if appropriate, further additives and/or adjuvants.

**6.** The process as claimed in claim 5, wherein the medicament prepared according to the process is intended for prevention and treatment of disorders in which the therapeutic use of inflammation inhibitors, analgesics, oxygen radical-deactivating agents and/or inhibitors or 5-lipoxygenase-induced degradation of arachidonic acid is indicated.

**7.** The process as claimed in claim 5 or 6, wherein the medicament prepared according to the process is intended for prevention and treatment of inflammatory disorders, in particular inflammatory rheumatic disorders, and/or pain.

**8.** The use of a compound of the formula I as defined in claim 1 or as obtained as claimed in one or more of claims 1 - 3, and/or the use of a physiologically acceptable acid-addition salt thereof, for the production of medicaments for prevention and treatment of the disorders mentioned in claims 6 and 7.

**Revendications**

**Revendications pour les Etats contractants suivants : AT, BE, CH, DE, FR, GB, IT, LI, LU, NL, SE**

**1.** 5-(3-alkyl-5-tert-butyl-4-hydroxyphényl)-2-amino-6H-1,3,4-thiadiazines de formule générale I

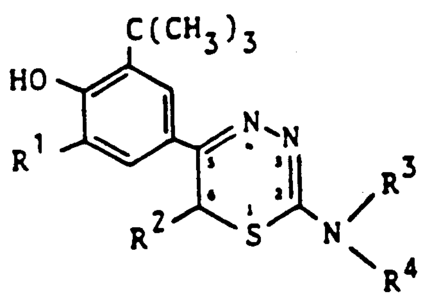

(I)

dans laquelle

$R^1$ représente un groupe alkyle à chaîne droite ou ramifiée ayant de 1 à 4 atomes de carbone et

$R^2$ représente un atome d'hydrogène ou un radical alkyle ayant de 1 à 3 atomes de carbone,

$R^3$ et $R^4$ représentent, indépendamment l'un de l'autre, un radical alkyle en $C_1$-$C_6$, alcényle en $C_3$-$C_6$ ou alcynyle en $C_3$-$C_6$ à chaîne droite ou ramifiée, à l'exception des inamines ou énamines, qui peuvent être substitués par le groupe phényle ou par un groupe phényle

substitué par un halogène ou par un groupe alkyle en $C_1$-$C_3$ ou alcoxy en $C_1$-$C_3$, par le groupe hydroxy ou un groupe acyloxy en $C_1$-$C_4$, et/ou dont les chaînes carbonées peuvent être interrompues par un hétéroatome sous forme d'oxygène ou de soufre, l'un des deux radicaux pouvant également représenter un atome d'hydrogène, ou

$R^3$ et $R^4$ forment, conjointement avec l'atome d'azote auquel ils sont liés, un cycle à 4-7 chaînons qui peut être une ou plusieurs fois substitué par un ou des groupes alkyle en $C_1$-$C_4$ et/ou contient, au lieu d'un des atomes de carbone formant le cycle, un autre hétéroatome sous forme d'oxygène, de soufre ou d'azote, ce dernier pouvant également porter, au lieu d'un atome d'hydrogène, un groupe alkyle ayant de 1 à 3 atomes de carbone ou un groupe phényle,

ainsi que leurs sels d'addition avec des acides physiologiquement acceptables.

**2.** Composés selon la revendication 1, caractérisés par au moins l'une des caractéristiques que $R^1$ représente le groupe tert-butyle ou méthyle ou $R^2$ représente un atome d'hydrogène ou le groupe méthyle ou $R^3$ et $R^4$ représentent indépendamment l'un de l'autre un groupe alkyle en $C_1$-$C_3$ ou alcényle en $C_3$-$C_4$, à chaîne droite ou ramifiée, à l'exception des énamines, qui peuvent être substitués par le groupe phényle ou hydroxy éventuellement substitué, ou dont la chaîne carbonée peut être interrompue par un atome d'oxygène, l'un des deux radicaux pouvant également représenter un atome d'hydrogène, ou $R^3$ et $R^4$ forment, conjointement avec l'atome d'azote auquel ils sont liés, un cycle à 5 ou 6 chaînons qui peut être substitué par un groupe alkyle en $C_1$-$C_2$ ou contient, au lieu d'un des atomes de carbone formant le cycle, un autre hétéroatome sous forme d'un atome d'oxygène ou d'azote, ce dernier pouvant également porter, au lieu d'un atome d'hydrogène, un groupe alkyle ayant un ou deux atomes de carbone.

**3.** Composés selon la revendication 1 ou 2, caractérisés en ce que $R^1$ représente un radical tert-butyle et simultanément $R^2$ représente un atome d'hydrogène ou le groupe méthyle, et $R^3$ et $R^4$ représentent, indépendamment l'un de l'autre, un groupe alkyle en $C_1$-$C_3$ ou alcényle en $C_3$-$C_4$, à chaîne droite ou ramifiée, à l'exception des énamines, qui peuvent être substitués par le groupe phényle, l'un des deux radicaux pouvant être également un atome d'hydrogène, ou $R^3$ et $R^4$ forment, conjointement avec l'atome d'azote auquel ils sont liés, un cycle pipéridine, 4-méthylpipéridine, pipérazine ou 4-méthylpipérazine.

**4.** Procédé pour la préparation des 5-(3-alkyl-5-tert-butyl-4-hydroxyphényl)-2-amino-6H-1,3,4-thiadiazines de formule I et de leurs sels d'addition avec des acides physiologiquement acceptables, selon une ou plusieurs des revendications 1 à 3, caractérisé en ce que l'on fait réagir une 2-halogéno-1-phénylalcanone de formule II

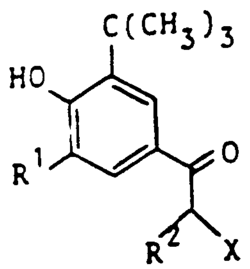

(II)

avec un thiosemicarbazide de formule III

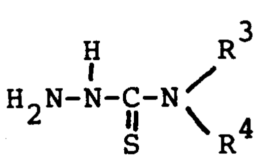

(III)

$R^1$, $R^2$, $R^3$, et $R^4$ ayant les significations données dans la revendication 1 et X représentant un atome d'halogène, pour aboutir aux composés de formule I, et soit on isole ceux-ci sous forme libre, soit on

les convertit, à l'aide d'acides appropriés, en sels d'addition physiologiquement acceptables.

**5.** Composés de formule I ainsi que leurs sels d'addition avec des acides physiologiquement acceptables, selon une ou plusieurs des revendications 1 à 3, pour utilisation en tant que médicaments.

**6.** Médicament caractérisé par une teneur en - ou consistant en - au moins un composé de formule I et/ou un de ses sels d'addition avec des acides physiologiquement acceptables, selon une ou plusieurs des revendications 1 à 3.

**7.** Médicament selon la revendication 6, pour la prévention et le traitement de maladies dans lesquelles est indiqué l'application thérapeutique d'anti-inflammatoires, d'analgésiques, d'agents inactivants des radicaux oxygénés et/ou d'inhibiteurs de la dégradation de l'acide arachidonique induite par la 5-lipoxygénase.

**8.** Médicament selon la revendication 6 ou 7, pour la prévention et le traitement de maladies inflammatoires, en particulier rhumatismales inflammatoires, et/ou d'états douloureux.

**9.** Procédé pour la fabrication d'un médicament selon une ou plusieurs des revendications 6 à 8, caractérisé en ce que l'on met sous une forme pharmaceutique appropriée au moins un composé de formule I et/ou au moins un sel d'addition avec un acide physiologiquement acceptable d'un tel composé, avec un véhicule physiologiquement acceptable et éventuellement d'autres additifs et/ou adjuvants.

**10.** Utilisation des composés de formule I et/ou de leurs sels d'addition avec des acides physiologiquement acceptables, pour la fabrication de médicaments selon les revendications 7 et 8.

**Revendications pour les Etats contractants suivants : ES, GR**

**1.** Procédé pour la préparation de 5-(3-alkyl-5-tert-butyl-4-hydroxyphényl)-2-amino-6H-1,3,4-thiadiazines de formule générale I

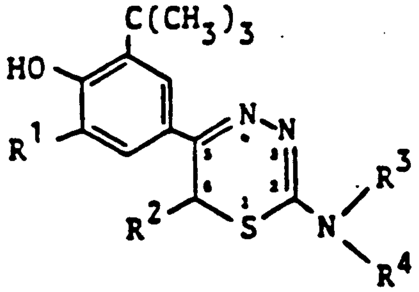

(I)

dans laquelle

$R^1$ représente un groupe alkyle à chaîne droite ou ramifiée ayant de 1 à 4 atomes de carbone et

$R^2$ représente un atome d'hydrogène ou un radical alkyle ayant de 1 à 3 atomes de carbone,

$R^3$ et $R^4$ représentent, indépendamment l'un de l'autre, un radical alkyle en $C_1$-$C_6$, alcényle en $C_3$-$C_6$ ou alcynyle en $C_3$-$C_6$ à chaîne droite ou ramifiée à l'exception des inamines ou énamines, qui peuvent être substitués par le groupe phényle ou par un groupe phényle substitué par un halogène ou par un groupe alkyle en $C_1$-$C_3$ ou alcoxy en $C_1$-$C_3$, par le groupe hydroxy ou un groupe acyloxy en $C_1$-$C_4$, et/ou dont les chaînes carbonées peuvent être interrompues par un hétéroatome sous forme d'oxygène ou de soufre, l'un des deux radicaux pouvant également représenter un atome d'hydrogène, ou

$R^3$ et $R^4$ forment, conjointement avec l'atome d'azote auquel ils sont liés, un cycle à 4-7 chaînons qui peut être une ou plusieurs fois substitué par un ou des groupes alkyle en $C_1$-$C_4$ et/ou contient, au lieu d'un des atomes de carbone formant le cycle, un autre

hétéroatome sous forme d'oxygène, de soufre ou d'azote, ce dernier pouvant également porter, au lieu d'un atome d'hydrogène, un groupe alkyle ayant de 1 à 3 atomes de carbone ou un groupe phényle,

ainsi que de leurs sels d'addition avec des acides physiologiquement acceptables, caractérisé en ce que l'on fait réagir une 2-halogéno-1-phénylalcanone de formule II

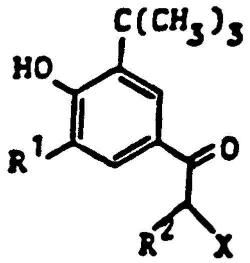

(II)

avec un thiosemicarbazide de formule III

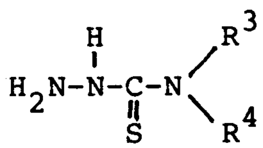

(III)

$R^1$, $R^2$, $R^3$ et $R^4$ ayant les significations données à propos de la formule 1 et X représentant un atome d'halogène, pour aboutir aux composés de formule I, et soit on isole ceux-ci sous forme libre, soit on les convertit, à l'aide d'acides appropriés, en sels d'addition physiologiquement acceptables.

**2.** Procédé selon la revendication 1, caractérisé en ce que l'on prépare des composés de formule I ayant au moins l'une des caractéristiques que $R^1$ représente le groupe tert-butyle ou méthyle ou $R^2$ représente un atome d'hydrogène ou le groupe méthyle ou $R^3$ et $R^4$ représentent indépendamment l'un de l'autre un groupe alkyle en $C_1$-$C_3$ ou alcényle en $C_3$-$C_4$, à chaîne droite ou ramifiée, à l'exception des énamines, qui peuvent être substitués par le groupe phényle ou hydroxy éventuellement substitué, ou dont la chaîne carbonée peut être interrompue par un atome d'oxygène, l'un des deux radicaux pouvant également représenter un atome d'hydrogène, ou $R^3$ et $R^4$ forment, conjointement avec l'atome d'azote auquel ils sont liés, un cycle à 5 ou 6 chaînons qui peut être substitué par un groupe alkyle en $C_1$-$C_2$ ou contient, au lieu d'un des atomes de carbone formant le cycle, un autre hétéroatome sous forme d'un atome d'oxygène ou d'azote, ce dernier pouvant également porter, au lieu d'un atome d'hydrogène, un groupe alkyle ayant un ou deux atomes de carbone.

**3.** Procédé selon la revendication 1 ou 2, caractérisé en ce que l'on prépare des composés de formule I dans lesquels $R^1$ représente un radical tert-butyle et simultanément $R^2$ représente un atome d'hydrogène ou le groupe méthyle, et $R^3$ et $R^4$ représentent, indépendamment l'un de l'autre, un groupe alkyle en $C_1$-$C_3$ ou alcényle en $C_3$-$C_4$, à chaîne droite ou ramifiée, à l'exception des énamines, qui peuvent être substitués par le groupe phényle, l'un des deux radicaux pouvant être également un atome d'hydrogène, ou $R^3$ et $R^4$ forment, conjointement avec l'atome d'azote auquel ils sont liés, un cycle pipéridine, 4-méthylpipéridine, pipérazine ou 4-méthylpipérazine.

**4.** Procédé pour la préparation des composés de formule I ainsi que de leurs sels d'addition avec des acides physiologiquement acceptables selon une ou plusieurs des revendications 1 à 3, pour utilisation en tant que médicaments.

**5.** Procédé pour la fabrication d'un médicament, caractérisé en ce que l'on met sous une forme pharmaceutique appropriée au moins un composé de formule I selon la définition donnée dans la revendication 1 et/ou au moins un sel d'addition avec un acide physiologiquement acceptable d'un tel composé et/ou au moins l'un des composés obtenus par le procédé selon une ou plusieurs des revendications 1 à 3, avec un véhicule physiologiquement acceptable et éventuellement d'autres

adjuvants et/ou additifs.

6. Procédé selon la revendication 5, caractérisé en ce que le médicament préparé selon le procédé est destiné à la prévention et au traitement de maladies dans lesquelles est indiquée l'application thérapeutique d'anti-inflammatoires, d'analgésiques, d'agents inactivants des radicaux oxygénés et/ou d'inhibiteurs de la dégradation de l'acide arachidonique induite par la 5-lipoxygénase.

7. Procédé selon la revendication 5 ou 6, caractérisé en ce que le médicament préparé selon le procédé est destiné à la prévention et au traitement de maladies inflammatoires, en particulier inflammatoires rhumatismales et/ou d'états douloureux.

8. Utilisation des composés de formule I selon la définition donnée dans la revendication 1 ou tels qu'obtenus selon une ou plusieurs des revendications 1 à 3, et/ou de leurs sels d'addition avec des acides physiologiquement acceptables, pour la fabrication de médicaments pour la prévention et le traitement des maladies mentionnées dans les revendications 6 et 7.